# EUROPEAN PATENT SPECIFICATION

(11) **EP 2 963 030 B1**
(45) Date of publication and mention of the grant of the patent: **13.12.2017**
(21) Application number: 13864793.8
(22) Date of filing: 18.01.2013
(51) Int. Cl.: C07D 311/32, A61K 31/352, A61P 11/00, A61P 11/14

(54) **6,8-SUBSTITUTED NARINGENIN DERIVATIVE AND USE THEREOF**
6,8-SUBSTITUIERTES NARINGENINDERIVAT UND VERWENDUNG DAVON
DÉRIVÉ DE LA NARINGÉNINE SUBSTITUÉ EN POSITIONS 6 ET 8 ET SON UTILISATION

(30) Priority: 19.12.2012 CN 201210557413
(43) Date of publication of application: 06.01.2016
(73) Proprietor: Sun Yat-Sen University, Guangzhou, Guangdong 510275 (CN)
(72) Inventor: SU, Weiwei, Guangzhou, Guangdong 510663 (CN); WANG, Yonggang, Guangzhou, Guangdong 510663 (CN); WU, Zhong, Guangzhou, Guangdong 510663 (CN)
(74) Representative: Sun, Yiming
(86) International application number: PCT/CN2013/070654
(87) International publication number: WO 2014/094370

(56) References cited:
- CN-A- 1 555 793
- JP-A- H09 301 915
- XINXIN CI ET AL: "Different Effects of Farrerol on an OVA-Induced Allergic Asthma and LPS-induced Acute Lung Injury", PLOS ONE, vol. 7, no. 4, 26 April 2012 (2012-04-26), page e34634, XP055231339, DOI: 10.1371/journal.pone.0034634
- FREDERIK ROELENS ET AL: "Subtle Side-Chain Modifications of the Hop Phytoestrogen 8-Prenylnaringenin Result in Distinct Agonist/Antagonist Activity Profiles for Estrogen Receptors [alpha] and [beta]", JOURNAL OF MEDICINAL CHEMISTRY, vol. 49, no. 25, 1 December 2006 (2006-12-01), pages 7357-7365, XP055231194, US ISSN: 0022-2623, DOI: 10.1021/jm060692n
- HSIEH Y-L ET AL: "Terpenoids and flavonoids from pseudotsuga wilsoniana", PHYTOCHEMISTRY, PERGAMON PRESS, GB, vol. 47, no. 5, 1 March 1998 (1998-03-01), pages 845-850, XP004293799, ISSN: 0031-9422, DOI: 10.1016/S0031-9422(97)00671-7
- DONG X ET AL: "Synthesis, biological evaluation and quantitative structure-activities relationship of flavonoids as vasorelaxant agents", BIOORGANIC & MEDICINAL CHEMISTRY, PERGAMON, GB, vol. 17, no. 2, 15 January 2009 (2009-01-15), pages 716-726, XP025893446, ISSN: 0968-0896, DOI: 10.1016/J.BMC.2008.11.052 [retrieved on 2008-11-24]
- XIANG RENDE ET AL.: 'Study on Chemical Constituents from Scutellaria barbata D. Don.' CHINESE HERBAL MEDICINES. vol. 13, no. 8, 1982, pages 9 - 12, XP008178117
- YANG JIANQIONG ET AL.: 'Study on Chemical Constituents from Reineckia carnea Kunth.' NATURAL PRODUCT RESEARCH AND DEVELOPMENT. vol. 22, 2010, pages 245 - 247, XP008178119
- GUO MIN FAN YING JIE ZHI ZU SHI JI DE HE CHENG: 'Synthetic Farrerol.' CHINESE JOURNAL OF PHARMACEUTICALS. vol. 8, no. 1, 1980, pages 41 - 42, XP008178118
- ZHANG JUNTIAN.: 'Studies on the pharmacology and chemistry of traditional Chinese and folk medicinal plants (one).' ACTA ACADEMIAE MEDICINAE SINICAE. vol. 1, no. 1, September 1979, pages 41 - 49, XP008178126
- LAETTIG, JENS ET AL.: 'Mechanism of inhibition of human secretory phospholipase A2 by flavonoids: rationale for lead design.' JOURNAL OF COMPUTER-AIDED MOLECULAR DESIGN. vol. 21, no. 8, 2007, pages 473 - 483, XP019528352
- YAIPAKDEE, P. ET AL.: 'Enzymatic halogenation of flavanones and flavones.' PHYTOCHEMISTRY vol. 57, no. 3, 2001, pages 341 - 347, XP055228451
- SVEN GESTER ET AL.: 'An efficient synthesis of the potent phytoestrogens 8-prenylnaringenin and 6-(1, 1-dimethylallyl) naringenin by europium (III)-catalyzed Claisen rearrangement.' TETRAHEDRON vol. 57, 2001, pages 1015 - 1018, XP004316531

## Description

### Technical Field

The present invention relates to a naringenin derivative, and particularly a 6,8-substituted naringenin derivative and use thereof in preparation of drugs for improving the functions of the respiratory system and especially drugs for relieving cough.

### Background Arts

Naringenin is the aglycone of naringin, and is widely present in the raw plant tissues including cherry blossom buds, plum blossom buds, peels of citrus fruit, and pummelo peels. Naringenin and naringin both have cough relieving effect. Naringenin is chemically stable in nature and remains as such after entering human body. Naringin is a glycoside of naringenin, which is formed by attaching a disaccharide at position 7 of naringenin and converted into naringenin by removing the disaccharide through hydrolysis in the presence of gastric acid and various enzymes after entering human body. Therefore, naringenin is the main form that exerts physiological effect in human body (Kazuo Ishii, Takashi Furuta, Yasuji Kasuya. Determination of naringin and naringenin in human plasma by high-performance liquid chromatography. Journal of Chromatography B, 683(1996); 225-229). The molecular structures of naringenin and naringin are as shown below:

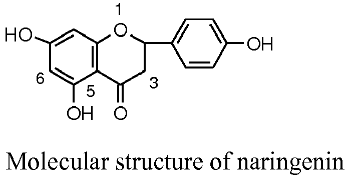

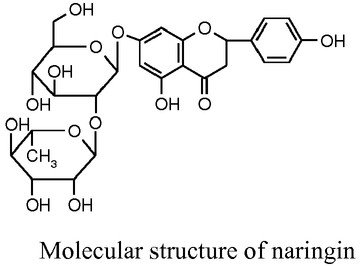

At present, codeine phosphate is clinically the most commonly used drug for reliving cough, which however results in addiction in clinic.

### Summary of the Invention

In the present invention, a 6,8-substituted naringenin derivative is developed by the researchers of the present invention after long-term studies, by further chemically modifying naringenin as lead compound. Moreover, a method for preparing the 6,8-substituted naringenin derivative is further provided. Experiments show that the naringenin derivative and salts thereof according to the present invention are obviously effective in improving the function of the respiratory system, for example, in respect of cough relieving, facilitating expectoration, and counteracting inflammation, and especially in relieving cough, and is useful in treatment of respiratory system diseases and disorders caused by bacteria and viruses.

The 6,8-substituted naringenin derivative described herein has a structure as shown in formula (I) below: 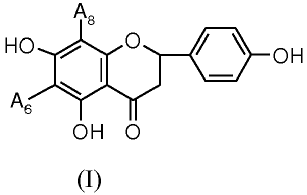 in which A₆ and A₈ are respectively selected from H, halogen, hydroxyl, an alkyl group with 1-3 carbon atoms, and a straight-chain alkenyl group, alcohol group, aldehyde group or carboxylic acid group containing 2-3 carbon atoms, provided that A₆ and A₈ preferably are not the same group.

The halogen is F, CI, Br or I.

The salt is preferably a potassium salt or a sodium salt, and may be prepared through conventional process.

The straight-chain alkenyl group, alcohol group, aldehyde group or carboxylic acid group containing 2-3 carbon atoms is preferably straight-chain propenyl, ethanol group, acetaldehyde group, or acetic acid group.

The alkyl group with 1-3 carbon atoms is preferably methyl, ethyl or propyl.

The present invention provides a 6,8-substituted naringenin compound as shown in general formula (I): 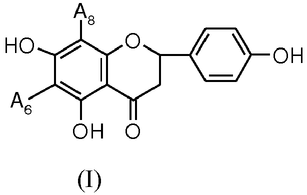 or a pharmaceutical acceptable salt thereof, characterized in that the compound is one of:
6-bromo-8-allylnaringenin,
6-allyl-8-bromonaringenin,
8-propyl-6-chloronaringenin,
6-chloro-8-allylnaringenin,
6-allyl-8-hydroxynaringenin,
6-propyl-8-chloronaringenin,
naringenin-6-acetaldehyde,
naringenin-8-acetic acid,
8-chloro-naringenin-6-acetic acid, or
6-chloro-naringenin-8-acetic acid.

The 6,8-substituted naringenin derivative according to the present invention can be prepared through a method comprising the specific steps of:
(1) preparing the intermediate compounds 8-allylnaringenin and 6-allylnaringenin of the derivative, specifically by subjecting a known compound 7-allyloxynaringenin as starting material to Claisen rearrangement in toluene, to obtain two isomers that are 8-allylnaringenin and 6-allylnaringenin respectively;
(2) as required by the position of the modifying group to be introduced, dissolving the intermediate compound 8-allylnaringenin or 6-allylnaringenin obtained in step (1) in ethanol, adding 10% Pd/C, hydrogenating for 20 hrs under normal pressure, filtering under suction, and purifying the filtrate by silica gel column chromatography eluting with a mixed solvent (2:1) of petroleum ether and ethyl acetate, to obtain a derivative substituted with a propyl group at position 8 or position 6 respectively;
(3) as required by the position of the modifying group to be introduced, dissolving the intermediate compound 8-allylnaringenin or 6-allylnaringenin obtained in step (1) in tetrachloromethane and dimethyl formamide, halogenating with equivalent amount of N-halosuccinimide, and after the reaction is complete, purifying the reaction solution by silica gel column chromatography eluting with petroleum ether : ethyl acetate 5:1, to obtain an allylnaringenin derivative substituted with halogen at position 6 or 8 respectively;
(4) as required by the position of the modifying group to be introduced, dissolving the derivative substituted with a propyl group at position 8 or 6 obtained in step (2) in tetrachloromethane and dimethyl formamide, halogenating with equivalent amount of N-halosuccinimide, and after the reaction is complete, purifying the reaction solution by silica gel column chromatography eluting with petroleum ether : ethyl acetate 5:1, to obtain a propylnaringenin derivative substituted with halogen at position 6 or 8 respectively;
(5) dissolving the intermediate compound 8-allylnaringenin or 6-allylnaringenin obtained in step (1) in dichloromethane and tetrahydrofuran, cooling the solution to -70°C in a dry ice-acetone bath under neat nitrogen atmosphere, then introducing ozone continuously for 2 hrs until the solution becomes blue colored and no discoloration occurs in ten minutes after the introduction of ozone is complete, whereupon introducing air for 15 min, then adding a reducing agent dimethyl sulfide, gradually heating to room temperature, mixing the resulting product with silica gel and purifying by silica gel column chromatography, to obtain a naringenin derivative substituted with an aldehyde group at position 8 or 6 respectively;
(6) dissolving the allylnaringenin derivative substituted with halogen at position 6 or 8 obtained in step (3) in ethyl acetate, then adding m-chloroperoxybenzoic acid, heating to reflux for 48 hrs, filtering under suction, concentrating the filtrate, and then purifying the residue by silica gel column chromatography, to obtain an allylnaringenin derivative substituted with a hydroxyl group at position 6 or 8 respectively;
(7) dissolving the naringenin derivative substituted with an aldehyde group at position 8 obtained in step (5) in ethanol, adding 1.1 eq of aminosulfonic acid, adding 1.1 eq of sodium chlorite dropwise at freezing point, and after complete reaction, mixing the resulting product with silica gel and purifying by silica gel column chromatography, to obtain a naringenin derivative substituted with chloro at position 6 and with a carboxylic acid group at position 8, and
   dissolving the naringenin derivative substituted with an aldehyde group at position 6 obtained in step (5) in ethyl acetate, adding 1.1 eq of aminosulfonic acid, adding 1.1 eq of sodium chlorite dropwise at freezing point, and after complete reaction, mixing the resulting product with silica gel and purifying by silica gel column chromatography, to obtain a naringenin derivative substituted with chloro at position 8 and with a carboxylic acid group at position 6; and
(8) dissolving the naringenin derivative substituted with an aldehyde group at position 8 obtained in step (5) in ethyl acetate, adding 1.1 eq of aminosulfonic acid, adding 1.1 eq of sodium chlorite dropwise at freezing point, and after the reaction is completed in 0.5 hr, mixing the product with silica gel and purifying by silica gel column chromatography, to obtain a naringenin derivative substituted with H at position 6 and with a carboxylic acid group at position 8.

The present invention encompasses a pharmaceutical composition which comprises the 6,8-substituted naringenin derivative as an active ingredient and a pharmaceutically acceptable carrier. Generally, the pharmaceutical composition of the present invention contains 0.1-95% by weight of the 6,8-substituted naringenin derivative according to the present invention.

The pharmaceutical composition containing the 6,8-substituted naringenin derivative according to the present invention may be prepared through a method generally known in the art. To this end, the 6,8-substituted naringenin derivative of the present invention may be combined with one or more solid or liquid excipients and/or adjuvants, if needed, to prepare drugs for human or veterinary use having suitable dosage forms for administration.

The 6,8-substituted naringenin derivative or the pharmaceutical composition containing the same according to the present invention may be administered in a unit dosage form through parental or non-parental routes, for example, oral, intramuscular, subcutaneous, nasal, transmucosal, transcutaneous, intraperitoneal, or rectal route of administration.

The 6,8-substituted naringenin derivative or the pharmaceutical composition containing the same according to the present invention may be administered through injection, including intravenous injection, intramuscular injection, hypodermic injection, intradermal injection and acupoint injection.

The dosage form for administration may be a liquid or solid dosage form. For example, the liquid dosage form may be a true solution, a colloid, a particulate, an emulsion, or a suspension dosage form. Other dosage forms include, for example, tablets, capsules, drop pills, aerosols, pills, powders, solutions, suspensions, suppositories, lyophilized powder injections, and others.

The 6,8-substituted naringenin derivative of the present invention may be prepared into an ordinary preparation, or into a sustained release preparation, a controlled release preparation, a targeting preparation, and various particulate delivery systems.

For example, where the unit dosage form is intended to be prepared into a tablet, various carriers generally known in the art may be widely used. Examples of the carriers include, for example, diluents and absorbents, such as starch, dextrin, calcium sulfate, lactose, mannitol, sucrose, sodium chloride, glucose, urea, calcium carbonate, kaolin, microcrystalline cellulose, aluminium silicate, and the like; wetting agent, such as water, glycerine, polyethylene glycol, ethanol, propanol, and the like; binders, such as starch paste, dextrin, syrup, honey, glucose solution, gum arabic, gelatin, carboxymethyl cellulose, methyl cellulose, shellac, potassium phosphate, polyvinyl pyrrolidone, and the like; disintegrants, such as dry starch, alginate, powdered agar, laminarin, sodium bicarbonate and citric acid, calcium carbonate, polyoxyethylene sorbitan fatty acid ester, sodium dodecylsulfonate, methylcellulose, ethylcellulose, and the like; disintegration inhibitors, for example, sucrose, glyceryl stearate, cacao butter, hydrogenated vegetable oil, and the like; absorption promoters, for example, quaternary ammonium salts, sodium dodecylsulfate, and the like; lubricants, for example, talc, silica, corn starch, stearate, boric acid, liquid paraffin, polyethylene glycol, and the like. The tablet may be further prepared into a coated tablet, for example, sugar coated tablet, tablet coated with films, tablet coated with enteric coating, double layered tablet, or multi-layered tablet.

For example, where the unit dosage form is intended to be prepared into a pill, various carriers generally known in the art may be widely used. Examples of the carriers include, for example, diluents and absorbents, such as glucose, lactose, starch, cacao butter, hydrogenated vegetable oil, polyvinyl pyrrolidone, kaolin, talc, and the like; binders, such as gum arabic, tragacanth, gelatin, ethanol, honey, rice paste or batter, and the like; disintegrants, such as powdered agar, dry starch, alginate, sodium dodecylsulfonate, methylcellulose, ethylcellulose, and the like.

For example, where the unit dosage form is intended to be prepared into a capsule, the 6,8-substituted naringenin derivative of the present invention as active ingredient is mixed with various carriers above, and the resulting mixture is encapsulated in a hard or soft gelatin capsule. Alternatively, the 6,8-substituted naringenin derivative of the present invention as active ingredient is prepared into a microcapsule, suspended in an aqueous medium to form a suspension, filled in a hard capsule, or prepared into an injection.

For example, the 6,8-substituted naringenin derivative of the present invention as active ingredient may be prepared into a preparation for injection, for example, a solution, a suspension, an emulsion, or a lyophilized powder injection. The preparation may be aqueous or non-aqueous, and contain one or more pharmaceutically acceptable carriers, for example, diluents, binders, lubricants, preservatives, surfactants or dispersants. For example, the diluents may be selected from the group consisting of water, ethanol, polyethylene glycol, 1,3-propylene glycol, ethoxylated isostearyl alcohol, polyoxyisostearyl alcohol, polyoxyethylene sorbitan fatty acid ester, and the like. Furthermore, in order to make the injection isotonic, a suitable amount of sodium chloride, glucose, or glycerine may be added to the preparation for injection. Further, conventional solubilizers, buffers, pH adjusting agents and others may be added as well. These adjuvants are those commonly used in the art. In addition, colorants, preservatives, fragrances, flavoring agents, sweeteners or other materials may be further added to the pharmaceutical preparation, if needed.

The dosage of the pharmaceutical composition containing the 6,8-substituted naringenin derivative according to the present invention depends on numerous factors, for example, the nature and severity of the diseases to be prevented or treated, the gender, age, body weight, disposition, and individual response of the patient or animal, route of administration, and dosage frequency etc. Therefore, the therapeutic dose in the present invention may vary over a wide range. The dosage of the pharmaceutical ingredient used in the present invention is generally known to persons of skill in the art, and may be appropriately adjusted according to the actual amount of the agent in the final preparation of the composition containing the 6,8-substituted naringenin derivative according to the present invention, to satisfy the requirement for therapeutic amount and thus accomplish the preventive or therapeutic purpose of the present invention. The daily dose of the 6,8-substituted naringenin derivative of the present invention is suitably in the range of 0.01-1000 mg/kg of body weight, and especially 1-100 mg/kg of body weight. The dose may be given in a single or several divided doses, for example, two, three or four divided doses, depending on the clinical experience of the prescribing physician and dosing regimen adopted in clinic.

It is confirmed through experiments that the 6,8-substituted naringenin derivative of the present invention are obviously effective in improving the function of the respiratory system in respect of cough relieving, facilitating expectoration, and counteracting inflammation, and most of the derivatives have a cough relieving effect superior to that of naringin or codeine. Particularly, the therapeutic efficacy of 8-n-propylnaringenin (the structure is shown in Example 1) is higher than naringenin. It can be inferred that the 6,8-substituted naringenin derivative of the present invention has a lower applicable dosage in clinic than naringin, and this class of substances are higher than naringin in therapeutic index and bioavailability. From the analysis of chemical structure-efficacy relationship of the agent, it is found that the derivative has a chemical structure similar to that of naringenin, but absolutely different from that of codeine phosphate, suffers no side effects of codeine phosphate in clinic such as addiction and resistance. Moreover, the molecular structure is simpler and more stable than naringin, and the synthesis process is simple. According, the derivative is suitable for scalable production in industry and has a promising prospect in development as drugs for relieving cough in placement of codeine phosphate or naringin in clinic.

### Examples

The following examples demonstrate the invention as long as they are covered by the claims.

### I. Preparation of 6,8-substituted naringenin derivative of the present invention

The preparation of the 6,8-substituted naringenin derivatives in Example 1-15 necessitates the intermediate compounds (18) and (19).

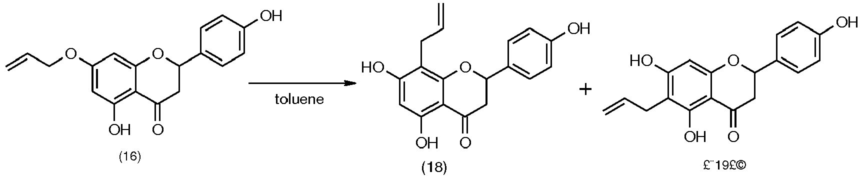

A known compound 7-allyloxynaringenin (16) as starting material was subjected to Claisen rearrangement in toluene, to obtain two isomers (18) and (19) that are 8-allylnaringenin and 6-allylnaringenin respectively.

The preparation process was as follows. The compound 7-allyloxynaringenin (16) (7.8 g) was placed in a 100 ml sealed glass reaction vessel and stood overnight in a sand bath at 180°C. The reactant was purified by silica gel column chromatography, to afford the compound (18) (1.8 g, yield 23%) and the compound (19) (1.25 g, yield 16%).

Compound (18): Mp. 190-191°C; Thin layer chromatography (TLC) Rf=0.45 (petroleum ether:ethyl acetate 2:1). High resolution mass spectrum *m*/*z* 312.0992[M]⁺; Molecular formula C₁₈H₁₆O₅.
¹H-NMR(400MHz,DMSO-*d6*): 2.752(1H,*dd*,*J₁*=17.2Hz, *J₂*=3.2Hz, H-3a), *δ*53.170(1H, *dd, J₁*=12.4Hz, H-3b),*δ*3.156(1H, *d, J*=6.4Hz,H-1"), *δ*4.890 (2H,*m*,H-3"),*δ*55.423(1H,*dd*,*J₁*=12.4Hz,*J₂*=2.8Hz,H-2),*δ*5.801(1H,*m*, H-2"),*δ*5.993 (1H,*s*, H-8),*δ*6.800(1H,d,*J*=8.4Hz,H-3', H-5'),*δ*7.302(1H, *d*, *J*=8.4Hz, H-2',H-6'), *δ*9.526(1H, *s*, -4'OH), *δ*10.734(1H,*s*,-70H), *δ*12.113(1H, *s*,-5OH).
¹³C-NMR(100MHz,DMSO-*d6*): *δ*197.09(C-4),*δ*164.86(C-7),*δ*161.84(C-5), *δ*160.38(C-9),*δ*157.99(C-4'),*δ*136.73(C-2"),*δ*129.65(C-1'),*δ*128.27(C-2',C-6'),*δ*15.63(C-3',C-5'),*δ*114.78(C-3"),*δ*105.54(C-8),*δ*102.23(C-10),*δ*95.74(C-6),*δ*78.54(C-2), *δ*42.33(C-3), *δ*26.73 (C-1").

Compound (19): Mp. 193-195°C; Thin layer chromatography (TLC) Rf=0.5 (petroleum ether:ethyl acetate 2:1). High resolution mass spectrometry *m*/*z* 312.0991[M]⁺; Molecular formula C₁₈H₁₆O₅.
¹H-NMR(400MHz,DMSO-*d6*): 2.681(1H, *dd, J₁*=16.8Hz, *J₂*=2.8Hz, H-3a), *δ*3.222(1H,*dd*,*J₁*=17.2Hz,*J₂*=4.4Hz,H-3b),*δ*3.182(1H,*d*, *J*=6.4Hz, H-1"), *δ*4.910(2H, *m*, H-3"),*δ*5.412(1H,*dd*,*J₁*=12.8Hz, *J₂*=2.8Hz, H-2), *δ*5.831(1H, *m,* H-2"),*δ*5.986 (1H,*s*, H-6), *δ*6.794(1H, *d, J*=8.4Hz, H-3',H-5'),*δ*7.311(1H,*d*, *J*=8.4Hz,H-2',H-6'), *δ*9.549(1H, *s*,-4'OH),*δ*10.732(1H,*s*,-70H),*δ*12.422(1H,*s*,-5OH).
¹³C-NMR(100MHz,DMSO-*d6*: *δ*196.94(C-4),*δ*164.83(C-5,C-9),*δ*158.16 (C-4'),*δ*136.56(C-2"),*δ*129.42(C-1'),*δ*128.72(C-2',C-6'),*δ*15.61(C-3',C-5'),*δ*14.75(C-3"),*δ*106.10(C-8),*δ*102.00(C-10),*δ*94.79(C-6),*δ*78.83(C-2),*δ*42.52(C-3),*δ*26.14(C-1").

### Example 1: 8-n-propylnaringenin (8-n-propyl-2,3-dihydro-5,7-dihydroxy -2-(4-hydroxyphenyl)chromen-4-one).

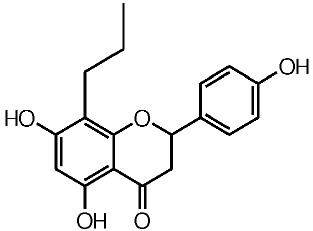

8-allylnaringenin (Compound 18) (1 g) was dissolved in ethanol (100 ml), and then 10% Pd/C (0.5 g) was added. The reaction system was hydrogenated for 20 hrs under normal pressure, and filtered under suction. The filtrate was concentrated and then the residue was purified by silica gel column chromatography eluting with petroleum ether:ethyl acetate=2:1, to afford a yellow solid (0.4 g). Mp. 195-197°C. Thin layer chromatography was performed with petroleum ether:ethyl acetate=1:1 as developing solvent. Rf=0.6. LRMS M-H=313.4, HPLC purity=99%, yield =40%.
¹H-NMR(400MHz, DMSO-*d6*): *δ*0.872(3H,*t*,*J*=7.2Hz;H-3"),*δ*1.441(2H,*q*, *J*=7.2Hz,H-2"),*δ*52.415(2H,*t*,*J*=8.4Hz,H-1"),*δ*2.651(1H,*dd*,*J₁*=16.8Hz,*J₂*=2.8Hz, H-3a),*δ*3.213(1H,dd,*J₁*=12.8Hz,*J₂*=16.8Hz,H-3b),65.386(1H,*dd,J₁*=12.8Hz, *J₂*=2.8Hz, H-2), *δ*5.945(1H,*s*,H-6),*δ*6.788 (2H,*d,J*=8.8 Hz,H-3',H-5'), *δ*7.301(2H,*d*, *J*=8.8Hz,H-2',H-6'),*δ*12.416(1H, s, 5-OH).
¹³C-NMR(100MHz,DMSO-*d6*): *δ*196.61(C-4),*δ*165.59(C-7),*δ*161.31(C-5), *δ*160.90(C-9),*δ*158.12(C-4'),*δ*129.52(C-1'),*δ*128.66(C-2',C-6'),*δ*15.58(C-3',C-5'), *δ*108.67(C-10),*δ*101.76(C-8), *δ*94.86(C-6),*δ*78.71(C-2),*δ*42.48(C-3),*δ*23.93(C-2"), *δ*22.11 (C-1"),*δ*14.40(C-3").

### Example 2: 6-n-propylnaringenin (6-n-propyl-2,3-dihydro-5,7-dihydroxy-2-(4-hydroxyphenyl)chromen-4-one).

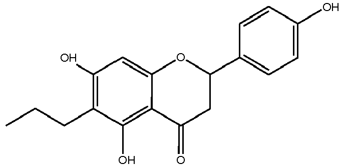

6-allylnaringenin (Compound 19) (1 g) was dissolved in ethanol (100 ml), and then 10% Pd/C (0.2 g) was added. The reaction system was hydrogenated for 20 hrs under normal pressure, and filtered under suction. The filtrate was concentrated and then the residue was purified by silica gel column chromatography eluting with petroleum ether:ethyl acetate=3:1, to afford a light yellow solid (0.4 g). Mp. 173-175°C. Thin layer chromatography was performed with petroleum ether:ethyl acetate=1:1 as developing solvent. Ref=0.6. MS M-H=313.2, HPLC purity=96%, yield =13%.
¹H-NMR(400MHz, DMSO-*d6*): *δ*0.822 (3H, *t*, *J*=7.2Hz; H-3"), *δ*1.417(2H,*q*, *J*=7.2Hz,H-2"),*δ*2.397(2H,*t*,*J*=7.2Hz,H-1"),*δ*2.733 (1 H,*dd,J₁*=17.2Hz, *J₂*=3.2Hz, H-3a),*δ*53.162(1H,*dd,J₁*=12.4Hz,*J₂*=16.8Hz,H-3b),*δ*5.408(1H,*dd,J₁*=12.4Hz, *J₂*=2.8Hz,H-2),*δ*5.976(1H,*s*,H-8),*δ*6.801(2H,*d*,*J*=8.4Hz,H-3',H-5'),*δ*7.304(2H,*d*, *J*=8.4Hz,H-2',H-6'),*δ*9.527(1H,*s*,4'-OH),*δ*10.630(1H,*s*,7-OH),*δ*12.112(1H,*s*,5-OH).
¹³C-NMR(100MHz, DMSO-*d6*): *δ*197.14(C-4),*δ*165.07(C-7), *δ*161.53(C-5), *δ*160.42(C-9),*δ*157.97(C-4'),*δ*129.79(C-1'),*δ*128.24(C-2',C-6'),*δ*115.65(C-3',C-5'), *δ*107.98(C-10),*δ*102.19(C-8),*δ*95.71(C-6),*δ*78.45(C-2),*δ*42.44(C-3),*δ*24.31(C-2"), *δ*22.33(C-1"),*δ*14.25(C-3").

### Example 3: 8-allyl-6-bromonaringenin (8-allyl-6-bromo-2,3-dihydro-5,7-dihydroxy-2-(4-hydroxyphenyl)chromen-4-one).

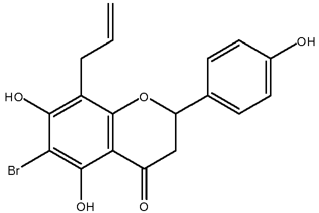

8-allylnaringenin (Compound 18) (0.7 g) was dissolved in tetrachloromethane (CCl₄) (50ml), dimethyl formamide (2ml) was added, and then N-bromosuccinimide (NBS) (1.05 eq) was added, and heated to reflux for 5 hrs. After cooling and concentration, the residue was directly mixed with silica gel and purified by silica gel column chromatography (petroleum ether:ethyl acetate=5:1), to obtain a light yellow solid (0.48 g). Mp. 203-205°C. Thin layer chromatography was performed with petroleum ether:ethyl acetate=3:1 as developing solvent. Rf=0.6. LRMS: M-H=391.2, 389.2; HPLC purity=89.22%, yield 55%.
¹H-NMR(400MHz,DMSO-*d6*): *δ*2.82(1H,*ddJ₁*=2.8 Hz, *J₂*=17.2Hz, H-3b), *δ*3.275(3H,*m*,H-1",H-3a),*δ*4.901(2H,*m*,H-3"),*δ*5.478(1H,*dd*, *J₁*=2.8Hz, *J₂*=12.8Hz, H-2),*δ*5.795(1H,*m*,H-2"),*δ*6.800(2H,*t*,H-3',H5'),*δ*7.305(2H,*d*,*J*=8.8Hz,H-2',H-6'), *δ*9.553(1H,*s*,H-4'OH),*δ1*0.334(1H,*s*,H-70H),*δ*12.840(1H,*s*,H-5OH).
¹³C-NMR(100MHz, DMSO-*d6*): *δ*197.59(C-4),*δ*160.59(C-5), *δ*159.33 (C-7), *δ*158.16(C-9),*δ*158.10(C-4'),*δ*136.15(C-2"),*δ*129.25(C-1'),*δ*128.36(C-2',C-6'),*δ*115.66(C-3',C-5'),*δ*115.16(C-3"),*δ*107.01(C-8),*δ*102.91(C-10),*δ*90.84(C-6),*δ*78.77(C-2), *δ*42.03(C-3),*δ*27.32(C-1").

### Example 4: 6-allyl-8-bromonaringenin (6-allyl-8-bromo-2,3-dihydro-5,7-dihydroxy-2-(4-hydroxyphenyl)chromen-4-one)

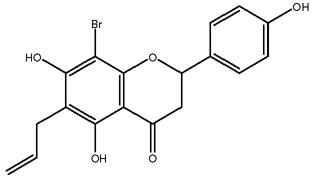

6-allylnaringenin (Compound 19) (0.7g) was dissolved in tetrachloromethane (50 ml), dimethyl formamide (2ml) was added, and then N-bromosuccinimide (NBS) (1.05eq) was added, and heated to reflux for 5 hrs until the raw materials were found to disappear as indicated by thin layer chromatography (TLC). After cooling and concentration, the residue was directly mixed with silica gel and purified by silica gel column chromatography (petroleum ether:ethyl acetate=5:1), to obtain a light yellow solid (0.7 g). Mp. 158-160°C. Thin layer chromatography was performed with petroleum ether:ethyl acetate=2:1 as developing solvent. Rf=0.6. LRMS: M-H=391.2, 389.2; HPLC purity=96.9%, yield 89%.
¹H-NMR(400MHz,DMSO-*d6*):*δ*2.843(1H,*dd,J₁*=3.2Hz,*J₂*=17.2Hz,H-3b),*δ*3.342(3H,*m*,H-1",H-3a),*δ*4.946(2H,*m*,H-3"),*δ*5.589 (1H,*dd,J₁*=2.8Hz,*J₂*=12.6Hz,H-2), *δ*5.840(1H,*m*,H-2"),*δ*6.814(2H,*t*,H-3',H5'),*δ*7.344(2H,*d*,*J*=8.8Hz,H-2',H-6'),*δ*9.584(1H,*s*,H-4'OH),*δ*10.297(1H,*s*,H-70H),*δ*12.458(1H,*s*,H-5OH).
¹³C-NMR(100MHz,DMSO-*d6*): *δ*197.35(C-4), *δ*160.63(C-5), *δ*160.05 (C-7), *δ*158.21(C-9),*δ*157.42(C-4'),*δ*135.98(C-2"),*δ*128.97(C-1'),*δ*128.59(C-2',C-6'),*δ*115.68(C-3',C-5'),*δ*115.12(C-3"),*δ*107.62(C-6),*δ*103.01(C-10),*δ*89.77(C-8),*δ*79.38(C-2), *δ*41.99(C-3),*δ*26.70(C-1").

### Example 5: 6-allyl-8-chloronaringenin (6-allyl-8-chloro-2,3-dihydro-5,7-dihydroxy-2-(4-hydroxyphenyl)chromen-4-one)

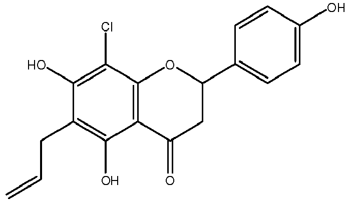

6-allylnaringenin (Compound 19) (0.5 g) was dissolved in tetrachloromethane (50 ml), dimethyl formamide (2ml) was added, and then N-chlorosuccinimide (NBS) (1.05 eq) was added, and heated to reflux for 5 hrs. After cooling and concentration, the residue was directly mixed with silica gel and purified by silica gel column chromatography (petroleum ether:ethyl acetate=5:1), to obtain a light yellow solid (0.3 g). Mp. 158-160°C. Thin layer chromatography was performed with petroleum ether:ethyl acetate=3:1 as developing solvent. Rf=0.6. LRMS: M-H=346.2; HPLC purity=95.5%, yield 54%.
¹H-NMR(400MHz,DMSO-*d6*): *δ*2.814(1H,*dd,J₁*=2.8Hz,*J₂*=17.4Hz,H-3b), *δ*3.342(3H,*m*,H-1",H-3a),*δ*4.947(2H,*q*,H-3"),*δ*5.584(1H,*dd*,*J₁*=2.8Hz,*J₂*=12.6Hz, H-2),*δ*5.850(1H,m,H-2"),*δ*6.820(2H,*d*, J=8.4Hz,H-3',H5'),*δ*7.344(2H,*d*,*J*=8.4Hz, H-2',H-6'),*δ*9.600(1H,*s*, H-4'OH),*δ*10.502(1H,*s*,H-7OH),*δ*12.393(1H,*s*,H-5OH).
¹³C-NMR(100MHz,DMSO-*d6*): *δ*197.36(C-4),*δ*159.81(C-5),*δ*159.27(C-7), *δ*158.31(C-9),*δ*156.35(C-4'),*δ*136.02(C-2"),*δ*128.92(C-1'),*δ*128.75(C-2',C-6'),*δ*115.72(C-3',C-5'),*δ*115.16(C-3"),*δ*107.56(C-8),*δ*102.73(C-10),*δ*99.60(C-6),*δ*79.57(C-2), *δ*42.09(C-3),*δ*26.62(C-1").

### Example 6:8-propyl-6-chloronaringenin (6-chloro-2,3-dihydro-5,7-dihydroxy-2-(4-hydroxyphenyl)-8-propylchromen-4-one).

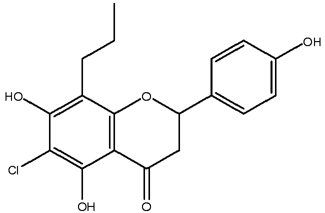

8-n-propylnaringenin (Example 1) (0.1 g) was dissolved in methanol (MeOH) (100 ml), and then 10% Pd/C (0.1 g) was added. The reaction system was evacuated, and then hydrogen was introduced, reacted for 3 hrs under 1 atm, and filtered. The filtrate was concentrated and then the residue was purified by silica gel chromatography, to obtain a light yellow solid (76 mg). Mp. 175-176°C. Thin layer chromatography was performed with petroleum ether:ethyl acetate=3:1 as developing agent. Rf=0.55. LRMS: M-H= 347.3, 349.4, HPLC purity=98.5%, yield 75%.
¹H-NMR(400MHz,DMSO-*d6*): *δ*0.893(3H,*t*,H-3"),*δ*1.457(2H,*q*,H-2"), *δ*2.523 (2H,*m*,H-1"),*δ*2.802(1H,*dd,J₁*=2.8Hz,*J₂*=17.2Hz, H-3b),*δ*3.351(1H,*q*,H-3a),*δ*5.570 (1H,*dd,J₁*=2.8Hz,*J₂*=12.8Hz,H-2),*δ*6.818(2H,*t*,H-3',H5'),*δ*7.340(2H,*d,J*=8.4Hz, H-2', H-6'),*δ*9.591 (1H,*s*,H-4'OH),*δ*10.366(1H,*s*,H-7OH),*δ*12.386(1H,*s*,H-5OH).
¹³C-NMR(100MHz,DMSO-*d6*):*δ*197.33(C-4),*δ*159.82(C-5),*δ*159.33(C-4'), *δ*158.27(C-9),*δ*155.96(C-7),*δ*128.95(C-1'),*δ*128.71(C-2',C-6'),*δ*115.68(C-3',C-5'), *δ*110.07(C-10),*δ*102.67 (C-8),*δ*99.53(C-6),*δ*79.46(C-2),*δ*42.11(C-3),*δ*24.43(C-2"), *δ*22.05(C-1"),*δ*14.28(C-3").

### Example 7: 8-allyl-6-chloronaringenin (8-allyl-6-chloro-2,3-dihydro-5, 7- dihydroxy-2-(4-hydroxyphenyl)chromen-4-one).

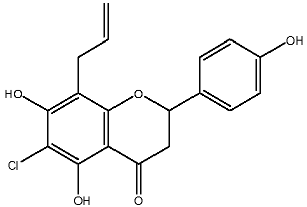

8-allylnaringenin (Compound 18) (0.5 g) was dissolved in tetrachloromethane (50 ml), dimethyl formamide (2 ml) was added, and then N-chlorosuccinimide (NCS) (1.05eq) was added and heated to reflux for 5 hrs. After cooling and concentration, the residue was directly mixed with silica gel and purified by silica gel column chromatography (petroleum ether:ethyl acetate=5:1), to obtain a light yellow solid (0.15 g). Mp. 215-216°C. Thin layer chromatography was performed with petroleum ether:ethyl acetate=3:1 as developing solvent. Rf=0.6. LRMS: M-H=346.2, HPLC purity=94.2%, yield 27%.
¹H-NMR(40OMHz,DMSO-*d6*): *δ*2.831(1H,*dd,J₁*=2.8Hz,*J₂*=17.2Hz,H-3b), *δ*3.283(3H,*m*,H-1",H-3a),*δ*4.908(2H,*m*,H-3"),*δ*5.483(1H,*dd,J₁*=2.8Hz,*J₂*=12.8Hz, H-2),*δ*5.810(1H,*m*,H-2"),*δ*6.804(2H,*t*,H-3',H5'),*δ*7.311(2H,*d,J*=8.8Hz,H-2',H-6'), *δ*9.562(1H,*s*,H-4'OH),*δ*10.553(1H,*s*,H-7OH),*δ*12.713(1H,*s*,H-5OH).
¹³C-NMR(100MHz,DMSO-*d6*):*δ*197.74(C-4),*δ*159.70(C-5),*δ*158.55(C-7),*δ*158.10(C-9),*δ*157.00(C-4'),*δ*136.15(C-2"),*δ*129.26(C-1'),*δ*128.36(C-2',C-6'),*δ*115.66 (C-3',C-5'),*δ*115.17(C-3"),*δ*107.01(C-8),*δ*102.63(C-10),*δ*100.45(C-6),*δ*78.79(C-2), *δ*42.15(C-3),*δ*27.18(C-1").

### Example 8:6-allyl-8-hydroxynaringenin (6-allyl-2,3-dihydro-5,7,8-Trihydroxy-2-(4-hydroxyphenyl)chromen-4-one)

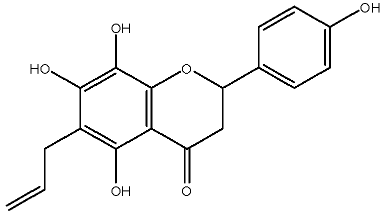

6-bromo-8-allylnaringenin (Example 3) (9 g) was dissolved in ethyl acetate (250 ml), and then m-chloroperoxybenzoic acid (10 g) was added, heated to reflux for 48, and filtered under suction. The filtrate was concentrated and then the residue was purified by silica gel column chromatography, to obtain a light yellow solid (280 mg). Mp. 95-100°C. Thin layer chromatography was performed with petroleum ether:ethyl acetate=1:1 as developing solvent. Rf=0.6. LRMS: M-H= 327.3, HPLC purity=86.5%, yield 29.6%.
¹H-NMR(400MHz,DMSO-*d6*):*δ*2.732(1H,*dd,J₁*=2.8Hz,*J₂*=17.2Hz,H-3b),*δ*3.259(3H,*m*,H-1",H-3a),*δ*4.921(2H,*q*,H-3"),*δ*5.436(1*H,dd,J₁*=2.8Hz,*J₂*=12.0Hz,H-2),*δ*5.844(1H,*d*,J=6.8Hz,H-2"),*δ*6.805 2H,*dd,J₁*=4.0Hz,*J₂*=8.4Hz,H-3',H5'),*δ*7.377 (2H,*d,J*=8.8Hz,H-2',H-6'),*δ*8.368(1H,*s*,H-8OH),*δ*9.568(1H,*s*,H-4'OH),*δ*9.877 (1H,*s*,H-7OH),*δ*12.070(1H,s,H-5OH).
¹³C-NMR(100MHz,DMSO-*d6*):*δ*197.07(C-4),*δ*158.09(C-4'),*δ*155.43(C-5), *δ*154.26(C-7),*δ*148.19(C-9),*δ*136.61(C-2"),*δ*129.60(C-1'),*δ*128.77(C-2',C-6'),*δ*125.17(C-8),*δ*115.52(C-3',C-5'),*δ*114.73(C-3"),*δ*105.86(C-10),*δ*101.60(C-6),*δ*78.94(C-2), *δ*42.83(C-3),*δ*26.47(C-1").

### Example 9:6-propyl-8-chloronaringenin (8-Chloro-2,3-dihydro-5,7-dihydroxy-2-(4-hydroxyphenyl)-6-propylchromen-4-one).

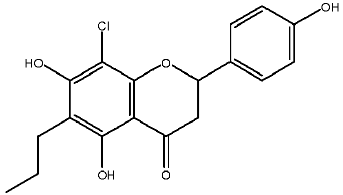

6-n-propylnaringenin(Example 2) (0.5 g) was dissolved in tetrachloromethane (50 ml), dimethyl formamide (2 ml) was added, and then N-chlorosuccinimide (NCS) (1.05 eq) was added and heated to reflux for 5 hrs. After cooling and concentration, the residue was directly mixed with silica gel and purified by silica gel column chromatography (petroleum ether:ethyl acetate=5:1), to obtain a yellow solid (0.1 g). Mp. 175-176°C. Thin layer chromatography was performed with petroleum ether:ethyl acetate=3:1 as developing solvent. Rf=0.6. LRMS: *m*/*z* 348.0759[M]⁺.
¹H-NMR(400MHz,DMSO-*d6*):*δ*0.838(3H,*t*=7.2Hz,H-3"),*δ*1.435(2H,*dd,J₁*=7.2Hz,*J₂*=14.8Hz,H-2"),*δ*2.498(2H,*m*,H-1"),*δ*52.816(1H,*dd,J₁*=2.8Hz,*J₂*=17.4Hz,H-3b),*δ*53.285(1H,*m*,H-3a),*δ*5.469(1H,*dd,J₁*=2.8Hz,*J₂*=12.8Hz,H-2),*δ*6.814(2H,*d*,J=8.8Hz,H-3',H5'),*δ*7.315(2H,*d,J*=8.4Hz,H-2',H-6'), *δ*9.594(1H,*s*,H-4'OH),*δ*10.422(1,*s*, H-7OH), *δ*12.708(1,*s*,H-5OH).
¹³C-NMR(100MHz,DMSO-*d6*):*δ*197.81(C-4),*δ*159.80(C-5),*δ*158.61(C-4'), *δ*158.09(C-7),*δ*156.68(C-9),*δ*129.40(C-1'),*δ*128.32(C-2',C-6'),*δ*115.69(C-3',C-5'),*δ*109.47(C-6),*δ*102.60 (C-10),*δ*100.42(C-8),*δ*78.70(C-2),*δ*42.26(C-3),*δ*24.84 (C-1"), *δ*22.27(C-2"),*δ*14.14(C-3").

### Example 10: 6-allyl-8-iodonaringenin (8-iodo-2,3-dihydro-5,7-dihydroxy-2-(4-hydroxyphenyl)-6-allylchromen-4-one)

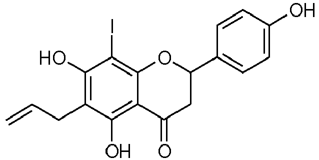

6-allylnaringenin (Compound 19) (0.5 g) was dissolved in tetrachloromethane (50 ml), DMF (2 ml) was added, and then N-iodosuccinimide (NIS) (1.05 eq) was added and heated to reflux for 3 hrs. The sample was directly mixed with silica gel and purified by silica gel column chromatography (petroleum ether:ethyl acetate=5:1), to obtain a light yellow solid (0.18 g). Mp. 218-220°C. Thin layer chromatography was performed with petroleum ether:ethyl acetate=3:1 as developing solvent. Rf=0.6. LRMS: M-H=437.2, HPLC purity=93.79%, yield 25%.
¹H-NMR(400MHz,DMSO-*d6*):*δ*(2H,*m*,H-3b),*δ*3.330(3H,*m*,H-1",H-3a),*δ*6.817(2H,*dd,J₁*=1.6Hz,*J₂*=8.4Hz,H-3',H5'),*δ*7.350(2H,*q*,H-2',H-6'),*δ*9.579(1H,*s*,H-4'OH),*δ*12.286(1H,*s*,H-5OH).

### Example 11: naringenin-8-acetaldehyde

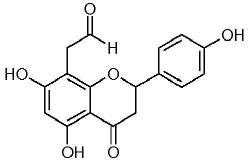

8-allylnaringenin (Compound 18) (2 g) was dissolved in dichloromethane (100 ml), and then tetrahydrofuran (THF) (20 ml) was added. The reaction vessel was evacuated, and then nitrogen was introduced. The solution was cooled to -70°C in a dry ice-acetone bath, then ozone was continuously introduced for about 2 hrs until the solution becomes blue colored and no discoloration occurs in ten minutes after the introduction of ozone is complete. Whereupon air was introduced for 15 min, then a reducing agent dimethyl sulfide (15 ml) was added and gradually heated to room temperature. The sample was mixed with silica gel and purified by silica gel column chromatography, to obtain a liquid (1 g), which was recrystallized in dichloromethane, to obtain a light yellow solid (0.3 g). Mp. 150-155°C. Thin layer chromatography was performed with petroleum ether:ethyl acetate=1:1 as developing solvent. Rf=0.5. LRMS: M-H=313.3, yield 15%.
¹H-NMR(400MHz,DMSO-*d6*):*δ*2.701(1H,*m*),*δ*3.243(1H,*m*),*δ*3.482(2H,*s*), *δ*5.450(1H,*m*),*δ*6.807(2H,*d,J*=8.4Hz),*δ*7.319(1H,*d,J*=8.4Hz),*δ*9.540,*δ*9.572(-4'OH), *δ*11.034(-70H),*δ*12.440(-5OH).
¹³C-NMR(100MHz,DMSO-*d6*): *δ*200.531,*δ*196.872,*δ*165.146, *δ*164.563, *δ*162.621,*δ*158.244,*δ*128.761,*δ*128.224,*δ*115.677,*δ*104.383,*δ*94.757,*δ*78.895, *δ*42.396,*δ*37.748,*δ*33.703.

### Example 12: naringenin-6-acetaldehyde

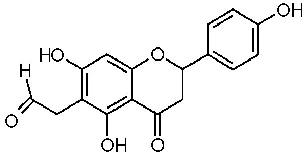

6-allylnaringenin (Compound 19) (12 g) was dissolved in ethyl acetate (100 ml). The reaction vessel was evacuated, and then nitrogen was introduced. The solution was cooled to -70°C in a dry ice-acetone bath, then ozone was continuously introduced for about 2 hrs until the solution becomes blue colored and no discoloration occurs in ten minutes after the introduction of ozone is complete. Whereupon air was introduced for 15 min, then a reducing agent dimethyl sulfide (15 ml) was added, gradually heated to room temperature and stirred for 2 hrs. The sample was mixed with silica gel and purified by silica gel column chromatography, to obtain a yellow solid (4.5 g). Mp. 178-180°C. Thin layer chromatography was performed with petroleum ether:ethyl acetate=1:1 as developing solvent. Rf=0.5. LRMS: M-H=313.3, yield 37%.
¹H-NMR(400MHz,DMSO-*d6*): *δ*2.708(1H,*m*),*δ*3.243(1H,*m*), *δ*3.514(2H,*s*), *δ*5.470(1H,*m*),*δ*6.799(2H,*d,J*=8.4Hz),*δ*7.321(1H,*d,J*=8.4Hz),*δ*9.573(-4'OH), *δ*11.028(-7OH), 12.489(-5OH).
¹³C-NMR(100MHz,DMSO-*d6*): *δ*200.447,*δ*197.059,*δ*165.164,*δ*162.067, *δ*161.735,*δ*158.221,*δ*129.314,*δ*128.777,*δ*115.651,*δ*100.310,*δ*94.809,*δ7*9.158, *δ*42.412,*δ*37.234,*δ*33.369.

### Example 13: 6-chloro-naringenin-8-acetic acid

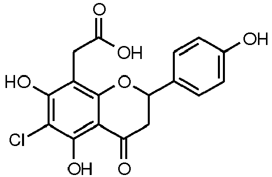

Naringenin-8-acetaldehyde (Example 11) (0.2 g) was dissolved in ethanol (100 ml), aminosulfonic acid (1.1 eq) was added, and then sodium chlorite (1.1 eq) was added dropwise at freezing point. The reaction was completed in 0.5 hr. The sample was mixed with silica gel and purified by silica gel column chromatography, to obtain a light yellow solid (0.05 g). Mp. 185-186°C. Thin layer chromatography was performed with dichloromethane:methanol=5:1 as developing solvent. Rf=0.5. LRMS: M-H=363.2, 365.2, HPLC purity=92.5%, yield 20%.
¹H-NMR(400MHz,DMSO-*d6*):*δ*2.868(1H,*m*),*δ*3.271(1H,*m*),*δ*3.451(2H,*m*),*δ*5.493(1H,*m*),*δ*6.809(2H,*d,J*=8.4Hz),*δ*7.3 08(1H,*d,J*=8.4Hz),*δ*9.561(-4'OH), *δ*10.784(-70H), *δ*12.725(-5OH).
¹³C-NMR(100MHz,DMSO-*d6*):*δ*197.624*δ*172.381,*δ*160.160,*δ*158.870, *δ*158.135,*δ*157.478,*δ*129.116,*δ*128.378,*δ*115.680,*δ*103.754,*δ*102.513,*δ*100.419,*δ*78.941,*δ*4 2.124,*δ*29.107.

### Example 14: 8-chloro-naringenin-6-acetic acid

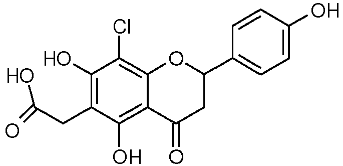

Naringenin-6-acetaldehyde (Example 12) (0.7 g) was dissolved in ethyl acetate (100 ml), aminosulfonic acid (1.1 eq) was added, and then sodium chlorite (1.1 eq) was added dropwise at freezing point. The reaction was completed in 0.5 hr. The sample was mixed with silica gel and purified by silica gel column chromatography, to obtain a light yellow solid (0.2 g). Mp. 185-186°C. Thin layer chromatography was performed with dichloromethane:methanol=5:1 as developing solvent. Rf=0.5. LRMS: M-H=363.2, 365.2, HPLC purity=97.34%, yield 24.5%.
¹H-NMR(400MHz,DMSO-d6): δ12.395(1"-OH), δ7.357(2', 6'-H, 2H,d, J=8.5Hz),δ6.831(3',5'-H,2H,d,J=8.5Hz),δ5.614(2-H,1H,*dd,J*=12.5Hz,J=3.0Hz), δ4.044(3-H, H, m, J=13.5Hz,J=7.0Hz), δ3.478(2"-H, 2H, S),δ2.844(3-H,H,*dd*, *J*=17.0Hz,J=3.0Hz).
¹³C-NMR(100MHz,DMSO-*d6*): *δ*197.285,*δ*172.380,*δ*160.245,*δ*159.502, *δ*158.328,*δ*156.780,*δ*128.878,*δ*128.746,*δ*115.737,*δ*104.299,*δ*102.581,*δ*99.537, *δ*79.620,*δ*42.014,*δ*28.487.

### Example 15: naringenin-8-acetic acid

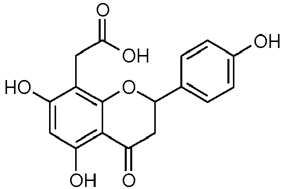

Naringenin-8-acetaldehyde (Example 11) (1 g) was dissolved in ethyl acetate, aminosulfonic acid (1.1 eq) was added, and then sodium chlorite (1.1 eq) was added dropwise at freezing point. The reaction was completed in 0.5 hr. The sample was mixed with silica gel and purified by silica gel column chromatography, to obtain a light yellow solid (0.2 g). Mp. 245-247°C. Thin layer chromatography was performed with dichloromethane:methanol=5:1 as developing solvent. Ref=0.5. LRMS: M-H=329.4, yield 19%.
¹H-NMR(400MHz,DMSO-*d6*):*δ*2.703(1H,*m*),*δ*3.271(1H,*m*),*δ*3.709(2H,*s*), *δ*5.416(1H,*m*),*δ*6.797(2H,*d,J*=8.4Hz),*δ*7.324(1H*,d,J*=8.4Hz),*δ*9.560(-4'OH), *δ*12.760(-5OH).

### II. Test on cough relieving effect of singly administered example compounds of the present invention in mice with cough induced from aqueous ammonia

### (i) Materials:

1. Test animals: male KM mice (available from Guangdong Medical Laboratory Animal Center, Certificate No.: SCXK (Guangdong) 2008-0002, No. 0113738).
2. Test groups and treatment with drugs:
   Blank control group: treatment with 50% (v/v) PEG400 in physiological saline.
   Treatment group with codeine phosphate as positive control - Codeine phosphate (Lot#: 20091212) is manufactured by Qinghai Pharmaceutical Co., Ltd. Before test, powdered codeine phosphate was added to 50% (v/v) PEG400 in physiological saline, to formulate a 0.47 µmol/ml solution.
   Example compounds: the powdered example compounds were formulated into 50% (v/v) PEG400 in physiological saline respectively, to give a 7.44 µmol/ml solution or suspension.
3. Reagents and instruments:
   YLS-8A cough and wheeze inducing instrument (a product available from Equipment Station, Shandong Academy of Medical Science).

Codeine phosphate (Qinghai Pharmaceutical Co., Ltd, Lot#: 20091212), 25% aqueous ammonia (Tianjin Guangcheng Chemical Reagent Co. Ltd., Lot#: 20110515), PEG400 (Tianjin Guangcheng Chemical Reagent Co. Ltd., Lot#: 20110308), 0.9% sodium chloride injection (Guangzhou Kelun Pharmaceutical Co. Ltd., Lot#: D1201104-1). Intragastric needle: customized in Laburatory Animal Center, Guangdong Test Center for Occupational Health.

### (ii) Test environment

Laboratory Animal Center, Sun Yat-sen University.

### (iii) Test method

Eligible male KM mice were randomly designated to a blank control group, and treatment groups with codeine phosphate as positive control and with example compounds, according to table 1.

The animals in each group were administered by intragastric gavage or intraperitoneal injection at a dosage of 0.2 ml/10 g of body weight, and placed in a cough inducing chamber 1 hr after administration. 1 ml of 25% concentrated aqueous ammonia was added to an atomizing cup and atomized for 15 s (at a rate of 0.15 ml/min). The coughs in 3 min were counted (during the typical cough, the mouth was opened and the head was lowered, which were accompanied by quivering of the neck and frequently a forward rushing).

Statistical analysis of data - Statistical analysis of all the data was conducted by SPSS 17.0 after the outliers were excluded by t test. Analysis of variance was performed by one-way ANOVA, multiple comparisons between the blank control group and each treatment group were carried out by Dunnett test, and the pairwise multiple comparisons between each treatment group and the codeine phosphate group were conducted by Duncan test.

### (iv) Test results - After statistical analysis, coughs of the animals in each group in 3 min after atomization are shown in Table 1.

**Table 1: Cough relieving effect of each compound in mice with cough induced from aqueous ammonia**

| Groups | Coughs | Cough relieving rate | P value compared with blank group |
|---|---|---|---|
| Example 1 | 6.2±3.5** | 65.11% | 0.000 |
| Example 2 | 12.4±4.6* | 30.16% | 0.028 |
| Example 3 | 11.2±6.2* | 37.28% | 0.019 |
| Example 4 | 11.0±4.1** | 38.03% | 0.005 |
| Example 5 | 11.8±7.3* | 33.49% | 0.047 |
| Example 6 | 9.1±3.9** | 49.06% | 0.001 |
| Example 7 | 10.5±5.8** | 40.85% | 0.009 |
| Example 8 | 10.2±2.6** | 42.64% | 0.002 |
| Example 9 | 10.7±4.2** | 39.82% | 0.004 |
| Example 10 | 12.2±4.0* | 31.24% | 0.016 |
| Example 11 | 11.4±6.5* | 35.78% | 0.026 |
| Example 12 | 9.3±4.3* | 47.59% | 0.002 |
| Example 13 | 7.4±3.4** | 58.43% | 0.000 |
| Example 14 | 9.3±3.9** | 47.59% | 0.001 |
| Example 15 | 8.3±5.8** | 53.31% | 0.002 |
| codeine phosphate | 10.8±3.5** | 39.60% | 0.003 |
| Blank group | 17.8±5.4 | | |

Hereinafter, the Preparation Examples provide the pharmaceu Tical Preparations containing the 6,8-substituted naringenin derivative obtained in Example 1 of the present invention.

**Preparation Example 1 - Tablet:**

| | |
|---|---|
| Compound of Example 1 | 10 mg |
| Lactose | 187 mg |
| Corn starch | 50 mg |
| Magnesium stearate | 3 mg |

Preparation method - The compound of Example 1, lactose, and corn starch were mixed, uniformly wetted with water, sieved, dried, and sieved again. Magnesium stearate was added, and then the mixture was tabletted. Each tablet weighed 250 mg.

**Preparation Example 2 - Capsule:**

| | |
|---|---|
| Compound of Example 1 | 10 mg |
| Lactose | 188 mg |
| Magnesium stearate | 2 mg |

Preparation method - The compound of Example 1, lactose, and magnesium stearate were mixed, sieved, and uniformly mixed in a suitable container, and then the mixture was filled in hard gelatin capsules. Each capsule weighed 200 mg.

**Preparation Example 3 - Ampoule:**

| | |
|---|---|
| Compound of Example 1 | 2 mg |
| Sodium chloride | 9 mg |

Preparation method - The Compound of Example 1 and sodium chloride were dissolved in a suitable amount of water for injection. The resulting solution was filtered and aseptically packaged in an ampoule.

**Preparation Example 4 - Nasal spray:**

| | |
|---|---|
| Compound of Example 1 | 80 mg |
| Sodium chloride | 8 mg |
| EDTA | 1 mg |
| Sodium phosphate buffer (pH 6.5) | 10 mg |
| Polysorbate | 10 mg |
| Double distilled water | 2 ml |

Preparation method - One ingredient was added to a suitable amount of double distilled water at a time with stirring until it was completely dissolved, followed by the addition of another ingredient. Water was added to give a volume of 2 ml, and then the solution was filtered through an aseptic filter, filled in a vial and spaced based on a suitable dose.

## Claims

1. A 6- and/or 8-substituted naringenin compound as shown in general formula (1): 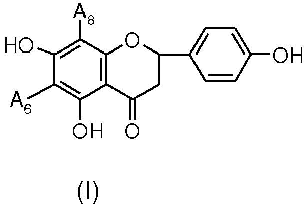 or a pharmaceutical acceptable salt thereof, **characterized in that** the compound is one of :
6-bromo-8-allylnaringenin,
6-allyl-8-bromonaringenin,
8-propyl-6-chloronaringenin,
6-chloro-8-allylnaringenin,
6-allyl-8-hydroxynaringenin,
6-propyl-8-chloronaringenin,
naringenin-6-acetaldehyde,
naringenin-8-acetic acid,
8-chloro-naringenin-6-acetic acid, or
6-chloro-naringenin-8-acetic acid.

2. The compound or a pharmaceutically acceptable salt thereof according to claim 1 for use for relieving cough.

3. The compound for use for relieving cough according to claim 2, wherein the compound is used as a single or in combination as active ingredient(s).

4. The compound according to claim 3 for use for relieving cough, **characterized in that** the active ingredients(s) is in a pharmaceutically acceptable dosage form, and the active ingredient is present in an amount of 0.1-95% by weight of the pharmaceutical preparation.

5. A 8-substituted naringenin compound as shown in general formula (1): 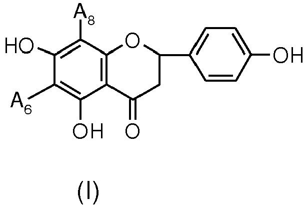 or a pharmaceutical acceptable salt thereof, for use for relieving cough, **characterized in that** the compound is 8-n-propylnaringenin.

## Patentansprüche

1. 6- und/oder 8-substituierte Naringenin-Verbindung, wie in der allgemeinen Formel (I) gezeigt: 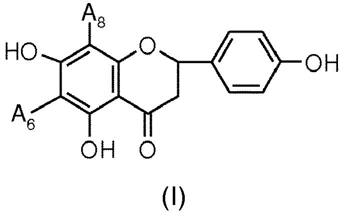 oder ein pharmazeutisch akzeptables Salz davon, **dadurch gekennzeichnet, dass** die Verbindung eines der Folgenden ist:
6-Brom-8-allylnaringenin,
6-Allyl-8-bromnaringenin,
8-Propyl-6-chlornaringenin,
6-Chlor-8-allylnaringenin,
6-Allyl-8-hydroxynaringenin,
6-Propyl-8-chlornaringenin,
Naringenin-6-acetaldehyd,
Naringenin-8-essigsäure,
8-Chlornaringenin-6-essigsäure, oder
6-Chlornaringenin-8-essigsäure.

2. Verbindung oder ein pharmazeutisch akzeptables Salz davon nach Anspruch 1 zur Verwendung zur Linderung von Husten.

3. Verbindung zur Verwendung zur Linderung von Husten nach Anspruch 2, wobei die Verbindung als ein einzelner oder in Kombination als aktive/r Inhaltsstoff/e verwendet wird.

4. Verbindung nach Anspruch 3 zur Verwendung zur Linderung von Husten, **dadurch gekennzeichnet, dass** der/die aktive/n Inhaltsstoff/e in einer pharmazeutisch akzeptablen Dosierungsform vorliegt/en und der aktive Inhaltsstoff in einer Menge von 0,1-95 % nach dem Gewicht des pharmazeutischen Präparates vorliegt.

5. 8-substituierte Naringenin-Verbindung, wie in der allgemeinen Formel (I) gezeigt: 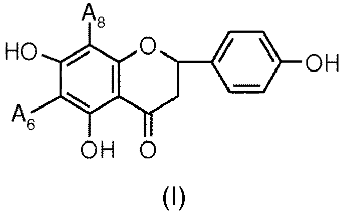 oder ein pharmazeutisch akzeptables Salz davon, zur Verwendung zur Linderung von Husten, **dadurch gekennzeichnet, dass** die Verbindung 8-n-Propylnaringenin ist.

## Revendications

1. Composé de naringénine substitué en positions 6 et/ou 8 tel que présenté dans la formule générale (1) : 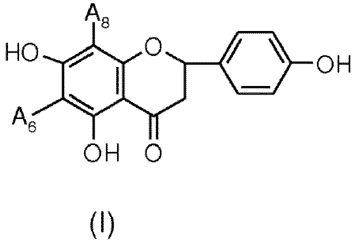 ou un sel pharmaceutiquement acceptable de celui-ci, **caractérisé en ce que** le composé est l'un de :
la 6-bromo-8-allylnaringénine,
la 6-allyl-8-bromonaringénine,
la 8-propyl-6-chloronaringénine,
la 6-chloro-8-allylnaringénine,
la 6-allyl-8-hydroxynaringénine,
la 6-propyl-8-chloronaringénine,
le naringénine-6-acétaldéhyde,
l'acide naringénine-8-acétique,
l'acide 8-chloro-naringénine-6-acétique, ou
l'acide 6-chloro-naringénine-8-acétique.

2. Composé ou un sel pharmaceutiquement acceptable de celui-ci selon la revendication 1, pour son utilisation pour soulager la toux.

3. Composé pour son utilisation pour soulager la toux selon la revendication 2, dans lequel le composé est utilisé seul ou en combinaison en tant qu'ingrédient(s) actif(s).

4. Composé selon la revendication 3 pour son utilisation pour soulager la toux, **caractérisé en ce que** l'ingrédient actif (les ingrédients actifs) se trouve(nt) sous une forme galénique pharmaceutiquement acceptable, et l'ingrédient actif est présent en une quantité de 0,1 à 95 % en poids de la préparation pharmaceutique.

5. Composé de naringénine substitué en position 8 tel que présenté dans la formule (1) : 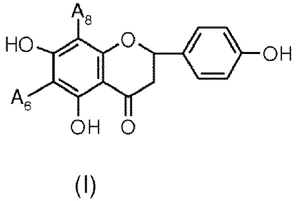 ou un sel pharmaceutiquement acceptable de celui-ci, pour son utilisation pour soulager la toux, **caractérisé en ce que** le composé est la 8-n-propylnaringénine.
